## Europäisches Patentamt

## European Patent Office

## Office européen des brevets

(19)

(11) Numéro de publication : **0 635 271 A1**

(12)

# DEMANDE DE BREVET EUROPEEN

(21) Numéro de dépôt : **94430005.2**

(22) Date de dépôt : **15.07.94**

(51) Int. Cl.[6] : **A61K 39/395**

(30) Priorité : **23.07.93 FR 9309382**

(43) Date de publication de la demande :
**25.01.95 Bulletin 95/04**

(84) Etats contractants désignés :
**AT BE CH DE DK ES FR GB GR IE IT LI LU MC NL PT SE**

(71) Demandeur : **Société Anonyme dite:**
**IMMUNOTECH S.A.**
**130, avenue de Lattre de Tassigny,**
**BP. 177**
**F-13276 Marseille Cédex 9 (FR)**

(72) Inventeur : **Brailly, Hervé**
**L'Amandière, Quartier du Clos**
**F-13360 Roquevaire (FR)**
Inventeur : **Montero-Julian, Félix Alejandro**
**Le Marie Louise Bat. C.**
**6, rue Marie-Louise**
**F-13008 Marseille (FR)**
Inventeur : **Klein, Bernard**
**30 rue de la Minée**
**F-44120 Vertou (FR)**

(74) Mandataire : **Rinuy, Santarelli**
**14, avenue de la Grande Armée**
**F-75017 Paris (FR)**

(54) **Nouveaux kits thérapeutiques anti-médiateurs protéiques, procédé de préparation et compositions pharmaceutiques les renfermant.**

(57)   Kits thérapeutiques anti-médiateurs protéiques à durée de vie courte et de taille suffisante pour disposer d'au moins trois sites anticorps, caractérisés en ce qu'ils renferment au moins trois anticorps reconnaissant trois épitopes distincts dudit médiateur protéique, au moins un desdits trois anticorps bloquant l'activité biologique dudit médiateur protéique, procédé de préparation et compositions pharmaceutiques les renfermant.

EP 0 635 271 A1

La présente demande concerne de nouveaux kits thérapeutiques anti-médiateurs protéiques, leur procédé de préparation et les compositions pharmaceutiques les renfermant.

L'interleukine 6 (IL-6) est impliquée dans de nombreux processus pathologiques. En particulier : l'IL-6 est le principal facteur de croissance in-vivo de plusieurs tumeurs dont le myélome multiple et la leucémie aiguë monoblastique, et est un facteur de croissance pour de nombreuses autres tumeurs dont le carcinome rénal et le sarcome de Kaposi. L'IL-6 est le principal médiateur induisant in-vivo la synthèse des protéines de phase aiguë par les hépatocytes, et est à ce titre impliqué dans la réponse inflammatoire systémique aiguë lors des chocs septiques. L'IL-6 participe à l'induction de la différenciation terminale et de la production d'immunoglobulines par les lymphocytes B et est donc impliquée dans l'activation oligoclonale et l'hyperglobulinémie observée dans les pathologies auto-immunes affectant le compartiment B, comme l'arthrite rhumatoïde et dans les infections par le VIH.

Pour toutes ces raisons, il serait souhaitable de disposer de méthodes et de produits permettant d'antagoniser l'activité de l'IL-6 in-vivo.

Différentes stratégies ont été décrites pour atteindre cet objectif.

Plusieurs drogues immunosuppressives ou anti-inflammatoires sont inhibitrices au niveau de la production des médiateurs de type cytokine. Cependant ces agents ne bloquent pas sélectivement l'activité d'un seul médiateur.

Une approche plus sélective consiste à bloquer le récepteur de l'interleukine 6 à l'aide d'un antagoniste spécifique. Un anticorps monoclonal liant le récepteur de l'IL-6 (IL-6R) de manière compétitive avec l'IL-6 a été décrit dans EP.A. 0409607. Cependant, le récepteur de l'IL-6 (IL-6R) existe sous forme soluble circulante à forte concentration dans le sérum, et présente une large distribution tissulaire, étant en particulier exprimé par les hépatocytes. Ces deux éléments limitent à priori l'efficacité et la spécificité d'un traitement par un anticorps anti-IL-6R

Une autre possibilité consiste à utiliser un agent liant l'IL-6 et empêchant celle-ci de se lier à son récepteur membranaire. Plusieurs anticorps monoclonaux anti-IL-6 utilisables en thérapeutique humaine ont été décrits (voir par exemple DE 3939706). L'un d'entre eux a été utilisé dans un essai clinique de traitement d'une leucémie plasmablastique en phase terminale (Klein B. et al, "Murine anti-interleukine-6 monoclonal antibody therapy for a patient with plasma cell leukemia" Blood 78 : 1198,1991). Cet essai a démontré que l'IL-6 contrôlait effectivement la prolifération tumorale. Cependant, le malade échappe au traitement du fait de l'accumulation d'IL-6 circulante sous la forme de complexes immuns avec l'anticorps anti-IL-6.

Pour sa part WO-A-92 01472 décrit l'utilisation de complexes d'immunoglobulines dans lesquels des immunoglobulines sont liées entre elles, pour bloquer dans l'organisme humain des cytokines, notamment sous forme multimère. Le nombre d'immunoglobulines formant de tels complexes est limité en raison de considérations de solubilité du complexe ainsi formé.

La présente invention fournit un produit capable de bloquer sélectivement une cytokine, notamment l'IL-6, in-vivo, par le moyen d'agents liant ladite cytokine en évitant par exemple dans le cas de l'IL-6, l'accumulation de cette dernière sous forme de complexes immuns de durée de vie très supérieure à celle de l'IL-6 libre, et plus généralement capable de bloquer certains médiateurs protéiques.

C'est pourquoi la présente invention a pour objet un kit thérapeutique anti-médiateurs protéiques à durée de vie courte et de taille suffisante pour disposer d'au moins trois sites anticorps, caractérisé en ce qu'il renferme au moins trois anticorps indépendants les uns des autres reconnaissant trois épitopes distincts dudit médiateur protéique, au moins un desdits trois anticorps bloquant l'activité biologique dudit médiateur protéique.

Par "médiateur protéique à durée de vie courte" l'on entend notamment des médiateurs circulants de nature glycoprotéique ou protéique, de préférence les cytokines, telles l'IL-1, l'IL-2, l'IL-4, l'IL-5, l'IL-6, l'IL-7, l'IL-9, l'IL-10, l'IL-11, l'Oncostatine-M, les facteurs de croissance hématopoïétiques tels le G-CSF, le GM-CSF, le M-CSF, le SCF, et tout particulièrement l'IL-6.

Ces médiateurs sont de taille suffisante pour disposer d'au moins trois sites anticorps distincts fonctionnels.

Par kit, l'on entend aussi bien l'association des anticorps dans des conteneurs différents, que l'association des anticorps dans le même conteneur. Ce kit comprend de préférence également des instructions d'utilisation des anticorps.

Pour sélectionner les anticorps et médiateur correspondant selon l'invention, on peut rechercher la capacité inhibitrice des anticorps vis-à-vis du médiateur, et vérifier que lesdits au moins trois anticorps agissent à des sites différents. De telles expérimentations figurent ci-après dans la partie expérimentale.

De manière surprenante, alors que la demi-vie plasmatique d'un médiateur, par exemple l'IL-6, complexé est sensiblement identique et de l'ordre de 12 à 24 heures dans le cas de l'utilisation d'un anticorps unique ou de deux anticorps, le seul ou l'un au moins des deux anticorps bloquant l'activité dudit médiateur, la demi-vie plasmatique de ce même médiateur complexé par un mélange de trois anticorps selon l'invention est analogue à celle du médiateur libre, c'est à dire de l'ordre de 20 mn.

Bien qu'il suffise qu'un desdits au moins trois anticorps selon l'invention soit capable de bloquer l'activité fonctionnelle du médiateur protéique, de préférence deux desdits au moins trois anticorps sont doués d'une telle propriété.

Les anticorps utilisés selon la présente invention sont une association de plusieurs anticorps avantageusement monoclonaux.

Les anticorps utilisés sont de préférence d'isotype IgG. On retient normalement des anticorps de souris, des anticorps de rat ou des anticorps humains de type IgG, et de préférence des anticorps de souris de type IgG2a ou des anticorps humains de type IgG1. Ces deux derniers types sont préférés du fait de l'affinité plus élevée de ces anticorps pour les récepteurs cellulaires pour les parties constantes des immunoglobulines (récepteurs FcRI).

Les anticorps mis en oeuvre ont de préférence une affinité pour l'antigène supérieure à $10^9$ litre/mole et particulièrement une affinité supérieure à $10^{10}$ litre/mole.

Les anticorps entrant dans la présente invention peuvent être d'origine animale, notamment murine, mais sont de préférence tout ou partiellement humanisés, par exemple grâce à l'une des techniques telles que celles décrites par "G. Winter et C. Milstein dans "Man-made antibodies", Nature Vol 349, pages 293-299.Lorsqu'ils sont partiellement humanisés, il s'agit au moins de leur partie Fc. L'utilisation en thérapeutique d'anticorps humanisés permet de réduire l'immunogénicité du produit injecté, ce qui peut se révéler avantageux pour des traitements répétés ou de longue durée. Il est particulièrement avantageux d'utiliser des anticorps chimériques dont les parties Fc proviennent d'un anticorps humain de type IgG1, pour simultanément diminuer l'immunogénicité du produit injecté et bénéficier de la plus grande affinité de ces anticorps pour les récepteurs Fc humains.

Par conséquent, une réalisation préférée de l'invention consiste en un kit contenant un ou plusieurs anticorps de préférence monoclonaux de souris ou de rat modifiés par génie génétique de manière à contenir une séquence peptidique provenant d'un anticorps d'origine humaine. Un exemple de telle réalisation de l'invention consiste en un kit contenant un ou plusieurs anticorps hybrides comprenant un fragment liant l'antigène provenant d'un anticorps monoclonal de souris ou de rat et au minimum une partie se liant au récepteur du fragment Fc d'origine humaine. Un autre type de réalisation de l'invention consiste en un kit selon l'invention contenant un ou plusieurs anticorps d'origine humaine établis à partir de clones de lymphocytes B humains, ou bien obtenus par recombinaison in-vitro d'éléments géniques du répertoire d'immunoglobulines humaines.

Les mélanges d'anticorps selon l'invention sont par exemple ceux constitués par les combinaisons suivantes :
- BE4, BE8 et AH65, BE4, AH64 et AH65, et
- BE4, BE8, AH64 et AH65.

Des dépôts des hybridomes BE4 et BE8 ont été effectués à la Collection Nationale de Culture de Microorganismes le 22 novembre 1989 respectivement sous les numéros I-911 et I-913.

Des dépôts des hybridomes AH64 et AH65 ont été effectués à la Collection Nationale de Culture de Microorganismes le 13 juillet 1993 respectivement sous les numéros I-1333 et I-1334.

Une description des anticorps BE4, BE8, AH64 et AH65 figure ci-après dans la partie expérimentale.

La présente demande a aussi pour objet un procédé de préparation des associations ou kits d'anticorps tels que définis ci-dessus caractérisé en ce que l'on associe au moins trois anticorps reconnaissant trois épitopes distincts dudit médiateur protéique, au moins un desdits trois anticorps bloquant l'activité biologique dudit médiateur protéique.

Comme on l'a vu, cette association peut concerner soit le mélange des anticorps dans le même récipient, soit des anticorps se trouvant dans des récipients séparés réunis dans le même emballage avec de préférence une notice d'instructions pour leur utilisation.

Dans des conditions avantageuses de mise en oeuvre du procédé ci-dessus décrit, les choix préférentiels indiqués ci-dessus pour les médiateurs et les anticorps s'appliquent également.

Les associations ou kits thérapeutiques ci-dessus sont doués de remarquables propriétés. Ils sont notamment capables d'antagoniser l'activité du médiateur contre lequel ils sont dirigés, chez l'homme ou l'animal, sans que l'individu traité n'échappe au traitement, c'est-à-dire sans que le traitement n'arrête de produire ses effets bénéfiques, par accumulation de complexes immuns.

Les propriétés ci-dessus sont illustrées ci-après dans la partie expérimentale et justifient l'application des associations d'anticorps ci-dessus au titre de médicaments.

C'est pourquoi la présente demande a aussi pour objet les médicaments caractérisés en ce qu'ils sont constitués par les kits ou associations d'anticorps définis ci-dessus.

Ces médicaments trouvent leur emploi par exemple pour agir sur des médiateurs dont il serait avantageux d'inhiber l'activité in-vivo dans différentes situations pathologiques. En particulier, cette méthode peut être mise à profit dans deux types de situations pathologiques : a) dans le cas de la production chronique à bas niveau d'une molécule de type cytokine normalement indétectable dans la circulation; b) dans le cas de la production massive aiguë d'une molécule de type cytokine.

Une réalisation de l'invention consiste donc à utiliser l'association d'anticorps décrite pour bloquer l'action d'une cytokine favorisant la croissance d'une tumeur. Des réalisations préférées consistent à inhiber in-vivo l'IL-1, l'IL-6, le GM-CSF dans des hémopathies malignes comme la leucémie aiguë myéloïde, le lymphome B, le myélome multiple, et l'IL-2, l'IL-7 et l'IL-9 dans les lymphomes T. En particulier, une réalisation préférée consiste à inhiber in-vivo l'action de l'IL-6 dans le myélome multiple, pour lequel l'IL-6 est un facteur essentiel de prolifération. Une autre réalisation préférée consiste à inhiber in-vivo l'action de l'IL-4 et de l'IL-5 dans les lymphomes Hodgkiniens. D'autres réalisations de l'invention concernent l'inhibition de médiateurs de type cytokine dans des tumeurs solides dont la prolifération est favorisée par ce médiateur. En particulier, des réalisations préférées consistent à inhiber in-vivo l'action de l'IL-6 dans le sarcome de Kaposi. Une autre réalisation préférée consiste à bloquer l'action de l'Oncostatine M dans le sarcome de Kaposi. Dans les situations aiguës, il est nécessaire de bloquer et d'éliminer rapidement le médiateur dont on souhaite inhiber l'action. Par suite la méthode décrite s'applique tout particulièrement à ces situations. Une réalisation envisagée consiste à inhiber l'IL-1 et l'IL-6 au cours des septicémies. Une réalisation préférée consiste à inhiber in-vivo l'action de l'IL-5 dans des syndromes à hyper-éosinophilie. Une réalisation tout particulièrement préférée consiste à inhiber l'action de l'IL-6 produite massivement lors des épisodes septiques qui touchent des patients souffrant d'une tumeur dont la croissance est favorisée par l'IL-6 comme c'est le cas en particulier dans le myélome multiple et pour les différentes hémopathies malignes citées précédemment ainsi que pour des patients HIV présentant un sarcome de Kaposi.

Ces applications ne sont citées qu'à titre d'illustration étant donné la variété de médiateurs protéiques à durée de vie courte visés par l'invention dont découle la variété d'actions pharmacologiques.

C'est pourquoi la présente demande a aussi pour objet une méthode d'antagonisation de l'activité d'un médiateur protéique à durée de vie courte et de taille suffisante pour disposer d'au moins trois sites anticorps, caractérisé en ce que l'on utilise une association d'au moins trois anticorps reconnaissant trois épitopes distincts dudit médiateur protéique, au moins un desdits trois anticorps étant en quantité suffisante pour bloquer (ou neutraliser) l'activité biologique dudit médiateur protéique.

Dans des conditions préférentielles de mise en oeuvre de la méthode ci-dessus décrite, on met en jeu de préférence les associations décrites ci-dessus pour antagoniser les médiateurs également décrits ci-dessus, notamment pour lutter contre les différentes situations pathologiques précédemment évoquées.

La présente invention a aussi pour objet les compositions pharmaceutiques caractérisées en ce qu'elles renferment l'un au moins des médicaments définis ci-dessus.

De manière générale, les compositions pharmaceutiques utilisables de préférence sont des préparations injectables. Les anticorps peuvent être conservés sous forme liquide ou solide, par exemple sous forme de lyophilisat. La réalisation préférée est celle pour laquelle les trois anticorps sont présents dans le même flacon. Dans une autre réalisation, les trois anticorps à injecter sont dans des flacons différents. Dans toutes les réalisations de l'invention, les trois anticorps peuvent être associés dans la préparation pharmaceutique à un ou plusieurs autres principes actifs ainsi qu'à des adjuvants avantageux quant aux qualités galéniques ou pharmacodynamiques de cette préparation.

La dose usuelle variable selon le sujet traité, l'affection en cause et les caractéristiques des anticorps constituant la combinaison injectée peut être, par exemple de 1 à 25 mg par jour par voie intraveineuse chez l'homme de l'association (BE4 + BE8 + AH65) administrés tous les jours pendant 10 à 20 jours lors d'un épisode septique survenant chez un patient atteint d'un myélome multiple.

Les exemples qui suivent illustrent la présente invention, sans toutefois la limiter.

## 1. Anticorps anti-IL-6

Des anticorps monoclonaux anti-cytokine peuvent être obtenus par immunisation de souris de diverses souches (par exemple Balb/c ou DBA2) avec des cytokines humaines recombinantes partiellement ou totalement purifiées et fusion de la rate de la souris immunisée avec un myélome murin du type de X63, selon la technique habituelle. Il est préférable de sélectionner les hybridomes produisant des anticorps anti-cytokine à l'aide de cytokine purifiée. Les anticorps utilisables dans un kit thérapeutique tel que décrit dans la présente invention doivent de plus présenter certaines propriétés particulières, c'est-à-dire (i) la capacité de au moins trois de ces anticorps à se lier simultanément à l'IL-6 (ii) la capacité d'au moins un de ces anticorps à bloquer l'activité biologique de l'IL-6. Les techniques expérimentales permettant de sélectionner des anticorps selon ces deux critères sont décrites ci-dessous.

Les anticorps monoclonaux de souris utilisés dans les exemples ci dessous sont désignés respectivement comme BE8, BE4, AH64 et AH65. Les hybridomes produisant les anticorps BE4 et BE8 ont été déposés et enregistrés à Paris à la Collection Nationale de Culture de Microorganismes (CNCM) le 27 novembre 1989 sous les numéros : BE8 : CNCM. N°I/913 ; BE4 : CNCM N°I/911. Les anticorps purifiés ont été obtenus commercialement auprès du CTS de Besançon (FRANCE). Une description complète de ces anticorps est donnée dans EP 0430193 ou DE 3939706. Les anticorps purifiés sont conservés stériles à +4°C.

Dans le cas des anticorps désignés comme AH64 et AH65, les hybridomes correspondant (respectivement D801D6M et D8812D3M) ont été cultivés selon les techniques habituelles. Les anticorps sont produits en ascite dans des souris nu/nu, puis purifiés sur colonne de protéine A (Pharmacia), selon les méthodes en vigueur. Les solutions d'anticorps purifié sont finalement diluées dans une solution saline isotonique de NaCl à 9g/l, stérilisées par filtration à 0,22µm et conservées à + 4°C. Les hybridomes produisant les anticorps AH64 et AH65 ont été déposés et enregistrés à Paris à la Collection Nationale de Culture de Microorganismes (CNCM) le 13 juillet 1993 sous les numéros AH64 : CNCM N° I-1333 ; AH65 : CNCM N° I-1334.

2. Caractérisation des anticorps utilisables dans un kit thérapeutique selon l'invention :

2.1. Caractérisation de la capacité inhibitrice des anticorps anti IL-6 utilisés :

Un kit thérapeutique constitué d'anticorps anti-IL-6 destiné à bloquer in-vivo l'activité biologique de l'IL-6 doit contenir au moins un anticorps anti-IL-6 qui inhibe l'activité biologique de ce médiateur. Cette capacité à inhiber l'activité de l'IL-6 peut être évaluée in-vitro à l'aide d'un essai biologique mesurant l'activité de l'IL-6.

L'activité biologique de l'Interleukine 6 est mesurée à l'aide de la lignée de plasmocytome murin IL-6-dépendant B9, décrite par L.A. Van Aarden (Eur. J Immunol. 17 : 1411, 1987). Cette lignée est cultivée dans un incubateur humidifié à 37°C sous 5% DE $CO_2$ (incubateur Narco), en milieu RPMI 1640 (formulation de référence dans le catalogue ATCC, page 354 de l'édition 1988), 2 mM L-glutamine, 1mM pyruvate, 50 UI/ml pénicilline et streptomycine, supplémenté en sérum de veau foetal 10% v:v (Gibco) et contenant 25 pg/ml d'Interleukine-6 humaine recombinante (Prepotech, Rocky Hill, NJ, USA). Tous les produits pour la culture sont fournis par Flow laboratories, Ecosse. La lignée est passée toutes les 48 heures, la concentration de la suspension cellulaire est maintenue inférieure à 5. $10^5$ cellules/ml. Le bioessai proprement dit est réalisé comme suit . La veille de l'essai la suspension cellulaire est centrifugée, lavée trois fois en RPMI 1640 et remise en culture à 2.$10^5$ cellules/ml dans le milieu précédent, mais en l'absence d'IL-6. Pour le bioessai, la suspension cellulaire est centrifugée, reprise en milieu complet sans IL-6 à $10^5$ cellules/ml et répartie dans une plaque de culture à 96 puits (Falcon) à 100 µl/puits soit 2.$10^4$ cellules/puits. Différentes dilutions calibrées d'IL-6 en milieu complet (0.5 à 50 pg/ml) ou d'IL-6 en présence de l'inhibiteur à tester sont distribuées dans la plaque de culture. Après 48 heures de culture, on distribue 0.25µCi de thymidine tritiée (Amersham) dans chaque puits, et la plaque est incubée pendant 8 heures à 37°C. Enfin, les cellules sont précipitées sur des filtres à l'aide d'un précipitateur (Skatron), les filtres sont transférés dans des tubes polypropylène où l'on ajoute 3 ml de liquide scintillant (Amersham) et la radioactivité incorporée dans le culot cellulaire est comptée dans un compteur bêta (Kontron).

On établit une gamme standard d'incorporation de radioactivité pour les différentes dilutions d'IL-6. La radioactivité incorporée pour un échantillon, ou en présence d'un inhibiteur de l'IL-6 est comparée à la valeur correspondante de la gamme standard. Pour chaque point, la mesure est réalisée sur trois puits.

**Expérience N° 1 :**

Les quatre anticorps utilisés (AH64, AH65, BE4, BE8) ont été caractérisés quant à leur capacité à inhiber la prolifération induite par l'Interleukine 6 de la lignée B9. L'essai a été réalisé avec une concentration fixe d'IL-6 calibrée (25 pg/ml) et différentes dilutions des anticorps testés, dans les conditions expérimentales définies précédemment. Pour chaque anticorps, la courbe de titration obtenue permet de mesurer la dose nécessaire (ED50) pour inhiber à 50 % la prolifération de la lignée B9 en présence de 25 pg/ml d'IL-6. Les résultats sont présentés dans le tableau A. Les quatre anticorps sont inhibiteurs de l'activité IL-6. L'anticorps AH65 est le plus efficace. Du point de vue de l'usage pharmaceutique, l'un quelconque des trois anticorps testés pourrait donc être utilisé, mais il serait plus avantageux pour réduire la dose efficace d'inclure l'anticorps le plus inhibiteur, tel AH65 dans une préparation associant plusieurs anticorps anti IL-6.

TABLEAU A

| ANTICORPS | DOSE EFFICACE 50 (M) |
|-----------|----------------------|
| AH65 | $3.3\ e^{-12}$ |
| AH64 | $2.3\ e^{-11}$ |
| BE8 | $1.1\ e^{-11}$ |
| BE4 | $1.2\ e^{-10}$ |

**Expérience N°2** : Effet synergique d'une combinaison d'anticorps inhibiteurs.

On a cherché à évaluer l'effet inhibiteur d'une combinaison d'anticorps anti-IL-6 bloquants sur la croissance de la lignée IL-6 dépendante B9. L'essai a été réalisé avec une concentration fixe d'IL-6 calibrée (25 pg/ml) et différentes dilutions soit de l'un des anticorps AH64 ou AH65, soit du mélange de ces deux anticorps en quantités égales. La capacité inhibitrice de chaque combinaison est évaluée par la dose totale d'anticorps inhibant à 50 % la prolifération de B9. On constate que la dose d'anticorps inhibitrice est plus faible lorsque l'on emploie un mélange que la dose d'anticorps pour chacun des anticorps constituant ce mélange. Ainsi, la dose inhibitrice du mélange AH65+AH64 en parts égales est trois fois plus faible que la dose inhibitrice d'AH65 et vingt fois plus faible que la dose inhibitrice d'AH64.

Ceci démontre l'effet synergique d'une combinaison d'anticorps inhibiteurs. Il est donc avantageux pour inhiber in-vivo l'activité de l'IL-6, d'utiliser une combinaison de plusieurs anticorps inhibiteurs.

### 2.2. Définition des épitopes de l'IL-6 liant les anticorps anti IL-6

### Enzymo-immunoessai de l'interleukine 6.

Dans un premier temps, on prépare des plaques de microtitration recouvertes d'anticorps monoclonal anti IL-6. Pour ce faire, une solution d'anticorps anti IL-6 purifié (10μg/ml en tampon phosphate 20 mM NaCl 0,15 M pH 7,5, tampon désigné comme PBS) est incubée pendant 24 heures à +4°C dans les puits d'une plaque de microtitration de polystyrène 96 puits (Nunc). La plaque est lavée à l'aide d'un laveur automatique (SLT, Salzburg, puis on ajoute 300μl/puits d'une solution de PBS contenant 10g/l d'albumine bovine (Boehringer, Manheim) pour une nouvelle incubation de 4 heures à température ambiante (tampon PBS-BSA). Parallèlement les différents anticorps à tester sont biotinés par réaction avec la biotine-N-hydroxy-succinimide-Ester (Boehringer Manheim), selon les instructions du fabricant. L'immunoessai proprement dit est effectué de la façon suivante :

Dans une plaque recouverte d'un anti-IL-6, on ajoute successivement 100μl de tampon PBS-BSA ou d'une solution d'IL-6 à 1 ou 10 ng/ml en PBS-BSA puis 100μl d'une solution d'anticorps biotiné à 1μg/ml dilué dans le même tampon. Dans certaines expériences, l'anticorps monoclonal anti IL-6 biotiné est ajouté dans une solution d'un troisième anticorps anti-IL-6 à 10 μg/ml. La plaque est incubée pendant 4 heures à +4°C, puis lavée 3 fois par une solution de lavage (PBS Tween 80 0,05% v:v) à l'aide d'un laveur automatique (SLT). Dans une deuxième étape, on ajoute dans chaque puits 200μl d'une solution de streptavidine couplée à la peroxydase (Jackson) diluée au 1/10000 en PBS/BSA. Après une incubation d'une heure à température ambiante, la plaque est lavée comme précédemment et la fixation de l'avidine-peroxydase et donc de l'anticorps biotiné est révélée par réaction colorimétrique en ajoutant dans chaque puits 200μL d'une solution de substrat contenant 1mg/ml de dichlorhydrate d'o-phénylènediamine (Sigma) et 10μl d' $H_2O_2$ à 30% dilué dans 10 ml de tampon phosphate-citrate 0.1 M pH 4,5. L'absorbance de chaque puits est lue à 492 nm dans un lecteur de microplaques (SLT, Salzburg). On peut considérer que deux anticorps lient des épitopes distincts de l'IL-6 si l'on obtient un signal positif pour l'immunoessai correspondant. Enfin trois anticorps peuvent lier simultanément l'IL-6 si la présence d'un troisième anticorps n'affecte pas le signal positif obtenu avec un couple d'anticorps donné.

**Expérience N° 3** :

On a préparé des plaques de micro-titration recouvertes avec les quatre anticorps anti-IL-6 AH65, AH64, BE8 et BE4 ainsi que quatre anticorps biotinés correspondants afin d'essayer toutes les combinaisons possibles dans un test de type immunométrique. L'essai est réalisé selon le protocole décrit précédemment. Les résultats sont exprimés en absorbance à 492 nm pour 1 ng/ml d'IL-6 incubée. On en déduit que les quatre anticorps se répartissent en trois groupes : AH65, BE4 et un épitope commun à AH64 et BE8 (tableau B). Ce résultat suggère que trois anticorps peuvent se lier simultanément à l'IL-6 : les combinaisons possibles sont AH64, AH65 et BE4 ou AH65, BE4 et BE8.

## TABLEAU B

| Traceur | Phase AH65 | Phase AH64 | Phase BE8 | Phase BE4 |
|---------|------------|------------|-----------|-----------|
| AH65 | − − | + + | + + | + + |
| AH64 | + + | − − | − − | + + |
| BE8 | + + | − − | − − | + + |
| BE4 | + | + + | + + | − − |

++ : densité optique >1.0

+ : densité optique <1.0

-- : négatif

**Expérience N°4 :**

Une deuxième expérience a été réalisée pour confirmer que les trois anticorps identifiés pouvaient effectivement se lier simultanément à l'IL-6. Pour cela, des immunoessais ont été effectués pour différentes paires d'anticorps compatibles en présence du troisième anticorps de chaque combinaison. On a constaté que le signal obtenu pour le couple (AH64, AH65) biotiné n'était pas affecté par la présence de l'anticorps BE4 à 10 μg/ml. De même, le signal obtenu pour le couple (BE4, BE8) n'est pas affecté par la présence de l'anticorps AH65. On en déduit que pour les deux combinaisons AH64+AH65+BE4 d'une part, AH65+BE8+BE4 d'autre part, les trois anticorps peuvent lier simultanément l'Interleukine 6 et définissent donc des épitopes distincts sur la molécule.

### 3. Pharmacocinétique de l'IL-6 radiomarquée in-vivo et effet de l'injection d'un ou plusieurs anticorps anti-IL-6:

L'injection de trois anticorps liant simultanément trois épitopes distincts de l'IL-6 permet d'éviter la stabilisation et l'accumulation de la molécule cible sous forme de complexes immuns. Au contraire, l'injection d'un seul ou de deux anticorps stabilise la molécule cible, et augmente sa durée de vie dans la circulation. Ces résultats expérimentaux démontrent la supériorité d'un kit thérapeutique constitué de trois anticorps liant trois épitopes distincts de la molécule cible.

### Marquage radioactif à l'iode 125 de l'interleukine-6.

L'interleukine-6 humaine (molécule recombinante produite dans E Coli. fournie par CLB Amsterdam) a été radiomarquée par la méthode à la chloramine T comme brièvement rappelé ci-après : dans un tube de réaction on ajoute successivement 2μg d'IL-6 à 100μg/ml en tampon phosphate salin isotonique pH 7.5 (tampon PBS), et 0.5 mCi d'une solution de $Na^{125}I$ (Amersham) à 100 mCi/ml. Puis la réaction est initiée par l'ajout de 10μl d'une solution de chloramine T (Merck) à 1mg/ml en PBS. Le mélange réactionnel est incubé pendant exactement 10 secondes à température ambiante (18-23°C) puis la réaction est arrêtée par l'ajout de 50μl d'une solution de glycyl-tyrosine (Bachem) à 10mM. Après une minute d'incubation, le mélange est dilué à un volume final de 0.5ml en tampon PBS contenant 10g/l d'albumine bovine (Boehringer) (PBS/BSA). Ce mélange est alors fractionné par tamisage moléculaire sur une colonne de Sephadex G50 Fine (Pharmacia) de 50 cm préalablement saturée en PBS-BSA. La colonne est éluée en PBS-BSA 0.2% au débit de 10 cm/heure et des fractions de 2 ml sont collectées. La radioactivité contenue dans chaque fraction est comptée dans un compteur gamma (Packard), et la concentration en IL-6 est déterminée à l'aide d'un immunoessai commercial (Immunotech). Les fractions contenant de l'IL-6 radiomarquée sont alors diluées en PBS/BSA de manière à obtenir une solution à $3.10^7$ cpm/ml. L'activité spécifique finale est obtenue par une nouvelle mesure de la concentration en IL-6 par immunoessai. L'activité spécifique se situe toujours entre $7,5.10^6$ à $17.10^6$ cpm/pmole soit 3 à $6.10^5$ cpm/ng.

Pharmacocinétique de l'IL-6 radiomarquée.

Les expériences de pharmacocinétique in-vivo sont réalisées sur des souris femelles Balb/c âgées de 19 semaines (fournies par Charles River) et maintenues en élevage dans les conditions usuelles. La veille de l'expérience les animaux reçoivent une dose de 50 µl de l'agent testé dilué en PBS-BSA stérile et apyrogène, par voie intraveineuse. Les animaux en contrôle sont traités par la solution isotonique seulement. Le jour de l'expérience, on injecte dans chaque souris par voie intraveineuse 50µl de la solution d'IL-6 radiomarquée soit exactement $10^6$ cpm. Après un temps donné (5, 10, 30, 60 minutes, 3, 6 et 24 heures), l'animal est sacrifié et pesé à l'aide d'une balance de précision. A l'aide d'une pipette Pasteur, on prélève selon la technique habituelle 1,5 ml environ du sang périphérique sur tube hépariné qui est immédiatement centrifugé pendant 5 minutes à 4000 G dans une centrifugeuse (Jouan) pour obtenir environ 500 µl de plasma. Les animaux sont ensuite disséqués et les organes suivants sont prélevés pour analyse : reins, poumon, coeur, rate, foie, intestin, thyroïde. La radioactivité présente dans le plasma et dans chaque organe est comptée dans un compteur gamma (Packard). Pour chaque organe, la radioactivité fixée est finalement exprimée en pourcentage de la dose totale injectée fixée par gramme. Enfin, les échantillons plasmatiques sont fractionnés par tamisage moléculaire afin de préciser la nature moléculaire des molécules radioactives présentes (voir ci-dessous). Pour chaque agent testé et chaque temps deux animaux sont traités en parallèle.

Chromatographie par tamisage moléculaire :

Le plasma des animaux auxquels on a injecté l'IL-6 radiomarquée est analysé par fractionnement sur une colonne de tamisage moléculaire. La chromatographie est réalisée sur une colonne Superdex Hi-Load 16/60 (Pharmacia) à l'aide d'un système de chromatographie de type FPLC (Pharmacia). On dépose un échantillon de 100 µl de plasma. La colonne est éluée en tampon Phosphate isotonique pH 7.50, à un débit de 2 ml/mn. Des fractions de 1ml sont collectées et la radioactivité présente dans chaque fraction est comptée dans un compteur gamma (Packard). La colonne a été préalablement calibrée en poids moléculaire à l'aide d'un mélange de protéines (Pharmacia), ce qui permet d'estimer le poids moléculaire des composants élués dans les différentes fractions.

**Expérience N° 5 :**

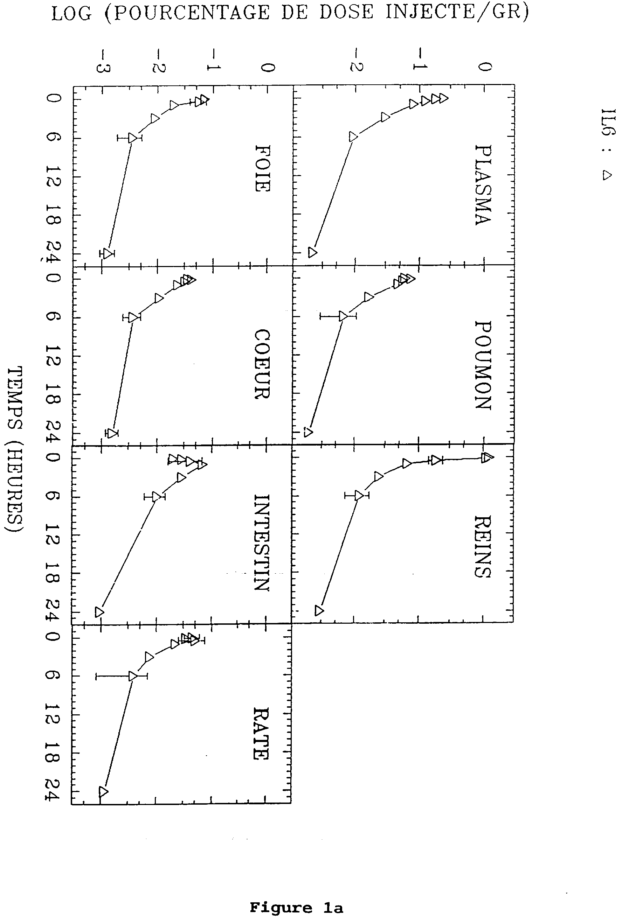

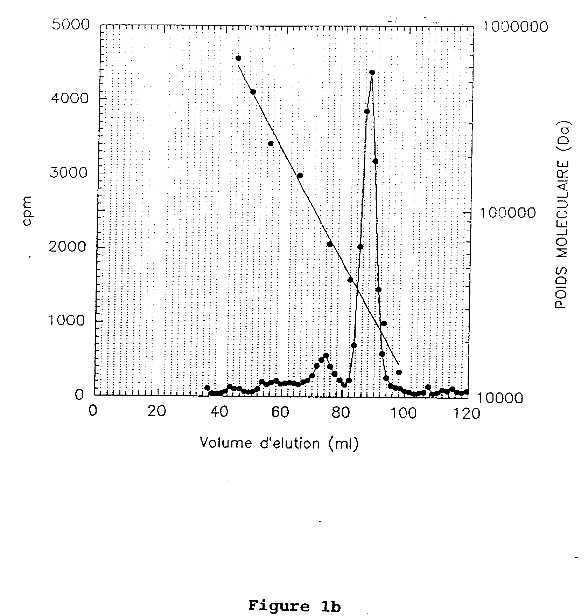

En premier lieu, on a étudié la pharmacocinétique de l'IL-6 humaine radiomarquée dans des souris Balb/c non traitées, selon le protocole décrit précédemment (injection de 100 µl de solution saline isotonique la veille de l'expérience). Les résultats sont présentés dans la figure la. La radioactivité présente dans le plasma décroît rapidement. On peut évaluer à 40-60 minutes la demi-vie de la molécule marquée dans le plasma. Le parallélisme des courbes de décroissance pour les différents organes testés indique qu'il n'y a pas d'accumulation spécifique de la molécule marquée. Un échantillon de plasma prélevé 10 minutes après injection de l'IL-6 radiomarquée a été fractionné par tamisage moléculaire. Le profil d'élution fait apparaître un pic majeur de radioactivité à 25 kD environ, ce qui correspond bien à l'IL-6 libre (Figure 1b).

**Expérience N° 6 :**

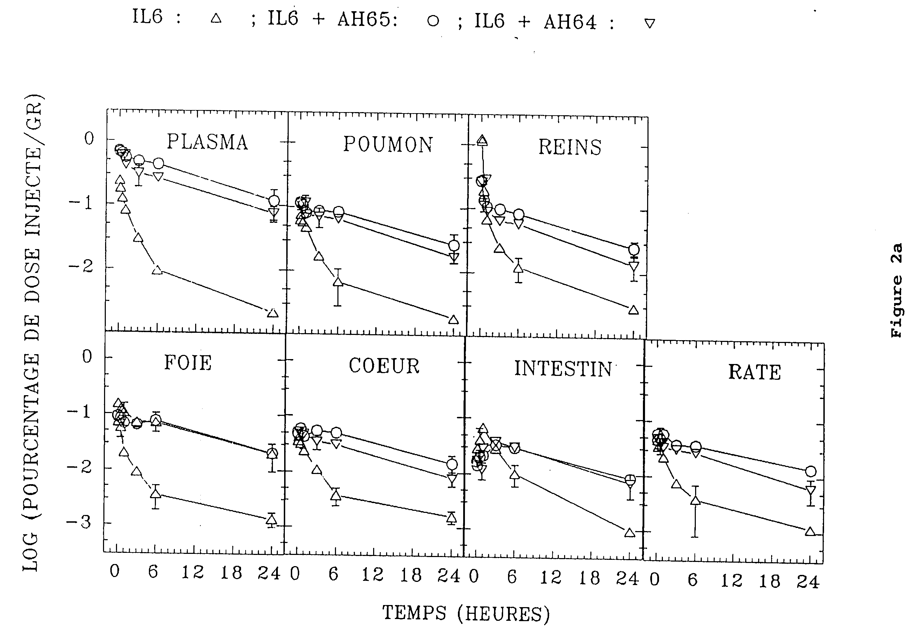

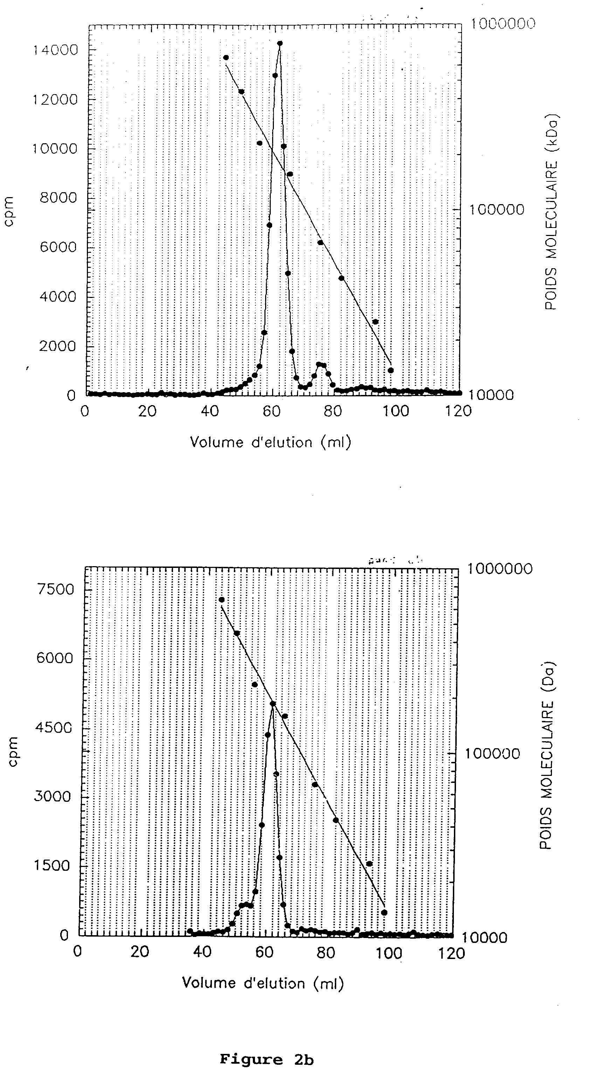

L'élimination de l'IL-6 radiomarquée a été analysée dans des souris traitées avec un anticorps anti-IL-6. La veille de l'expérience, on a injecté aux animaux 5 µg de l'un des quatre anticorps AH64, AH65, BE4 ou BE8. La pharmacocinétique de l'IL-6 humaine radiomarquée a été étudiée comme précédemment. Les résultats sont présentés dans la Figure 2a. La radioactivité présente dans le plasma ainsi que dans les différents organes testés décroît beaucoup plus lentement. On peut estimer la demi-vie de la molécule marquée dans le plasma entre 12 et 24 heures, selon l'anticorps anti-IL-6 utilisé. Afin de confirmer la nature de la radioactivité présente à différents temps après l'injection d'IL-6, des échantillons de plasma ont été fractionnés par tamisage moléculaire, dans les conditions décrites précédemment. A titre d'exemple, les profils d'élution aux temps 10 minutes et 6 heures sont présentés en Figure 2b, pour des animaux traités avec l'anticorps AH65. Aux deux temps indiqués ci-dessus, la radioactivité est éluée dans un pic majoritaire qui correspond à un poids moléculaire d'environ 180 Kd. Cette expérience confirme que la forme plasmatique prépondérante est l'IL-6 radiomarquée liée à un anti-IL-6 dans un complexe monovalent.

Dans des animaux traités avec un anti-IL-6 l'Interleukine 6 est donc stabilisée et s'accumule sous forme de complexes immuns.

**Expérience n°7 :**

La pharmacocinétique de l'IL-6 radiomarquée a été analysée dans des animaux traités avec des combinaisons de plusieurs anticorps anti IL-6 liant des épitopes différents de la molécule, les traitements suivants ont été appliqués : AH64+AH65, BE4+BE8, AH65+BE4+BE8, AH64+AH65+BE4+BE8. La combinaison de quatre anticorps correspond en fait à trois épitopes distincts de la molécule d'IL-6.

Pour chaque traitement, la dose totale d'anticorps injecté était de 5μg. Une même dose de chaque anticorps a été utilisée dans les différentes combinaisons : 2,5μg pour l'association de deux anticorps, 1,66μg pour l'association de trois anticorps, 1,25μg pour l'association de quatre anticorps. La cinétique d'élimination de l'IL-6 a été suivie comme précédemment.

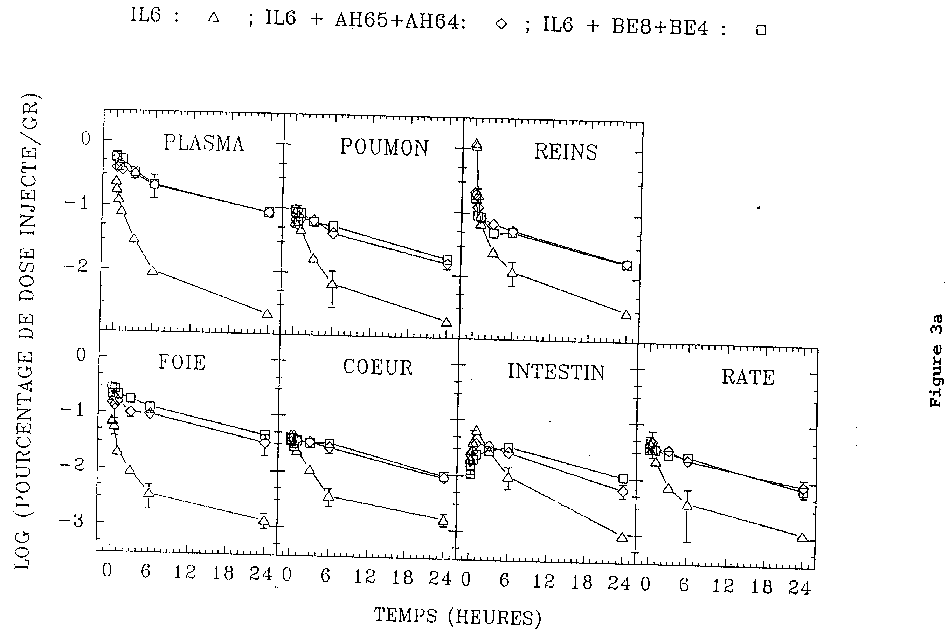

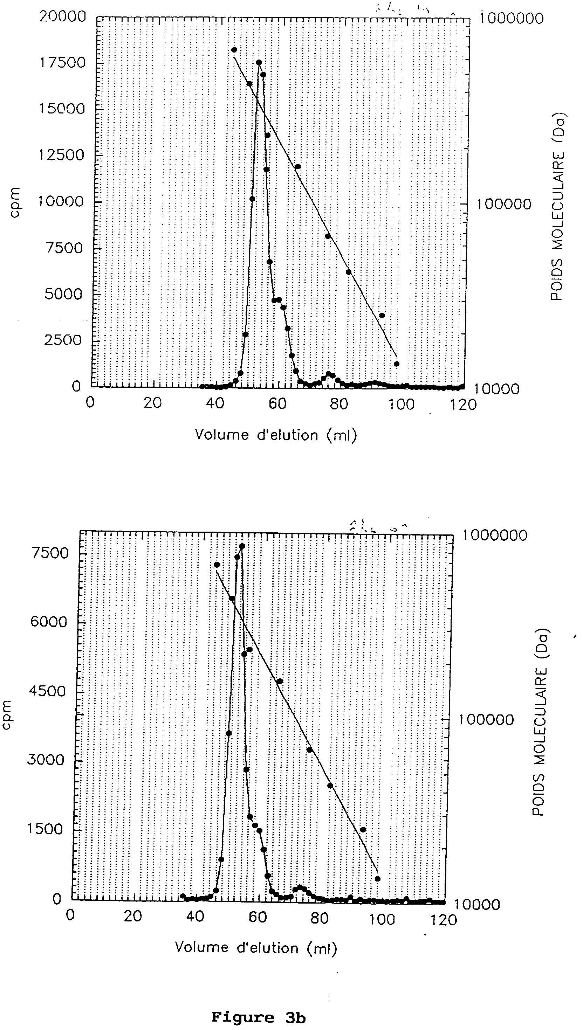

Dans le cas d'un traitement par deux anticorps, la vitesse d'élimination de la radioactivité est proche de celle observée dans les animaux traités par un seul anticorps monoclonal. Les résultats sont similaires pour les deux couples d'anticorps utilisés. Le fractionnement par tamisage moléculaire d'échantillons plasmatiques prélevés respectivement 15 minutes et deux heures après l'injection d'IL-6 radiomarquée dans des animaux traités par la combinaison AH64+AH65 permet de constater que les complexes immuns divalents (IL-6 et deux anticorps) sont bien la forme prépondérante de l'IL-6 circulante (voir Figures 3a et 3b).

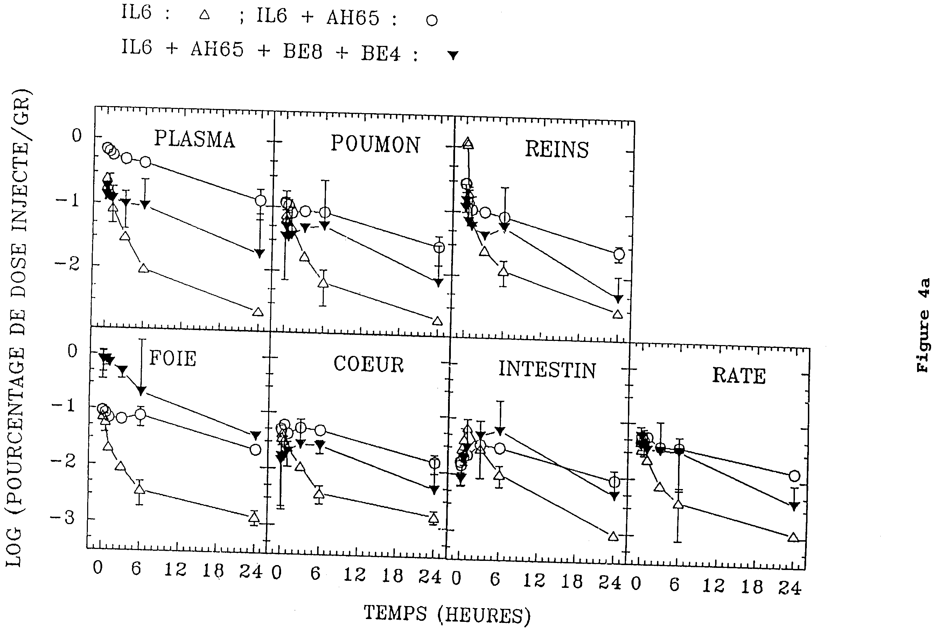

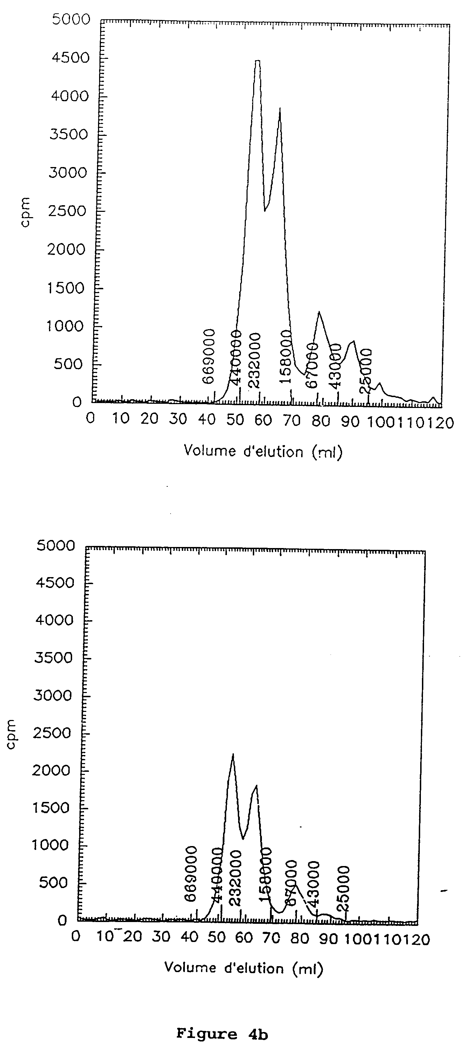

Dans le cas d'un traitement par trois anticorps (tels que AH65+BE4+BE8) liant trois épitopes distincts de l'IL-6, la vitesse initiale d' élimination de l'IL-6 radiomarquée est proche de celle de l'IL-6 libre. L'analyse de la distribution tissulaire de la radioactivité fait apparaître une accumulation spécifique transitoire de l'IL-6 dans le foie, la rate et l'intestin qui suggère une modification de la voie d'élimination (voir Figure 4a). Le fractionnement du plasma montre que les complexes trivalents (IL-6 et trois anticorps) sont immédiatement éliminés. Ces complexes ne sont pas détectables 10 minutes après l'injection . Les formes circulantes prépondérantes sont les formes monomériques et dimériques, ce qui confirme que les complexes trivalents sont éliminés préférentiellement. La présence de complexes respectivement monovalents et divalents peut s'expliquer par la réactivité partielle du traceur avec les anticorps utilisés, du fait de l'hétérogénéité introduite par l'étape de iodation (voir Figure 4b).

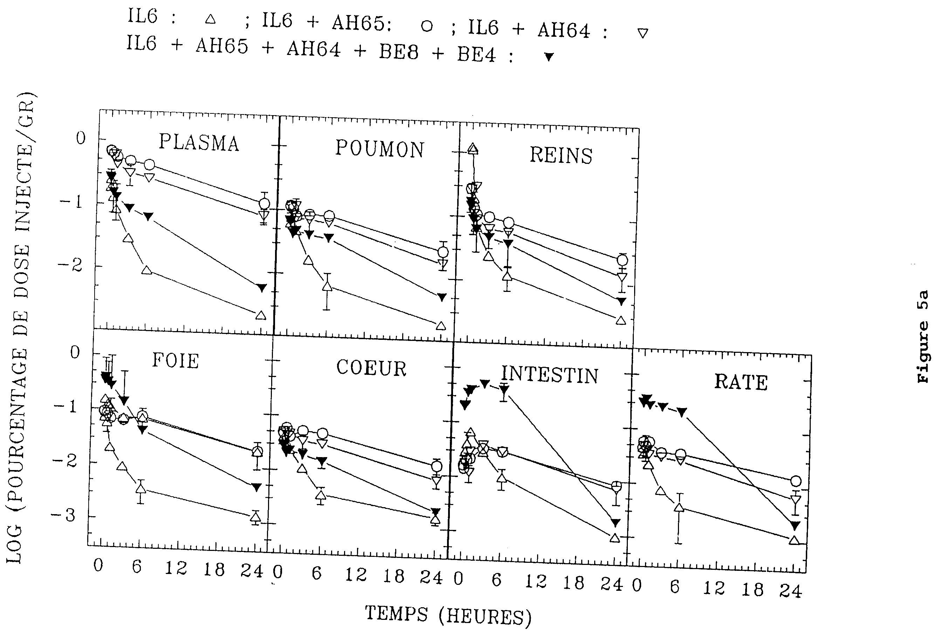

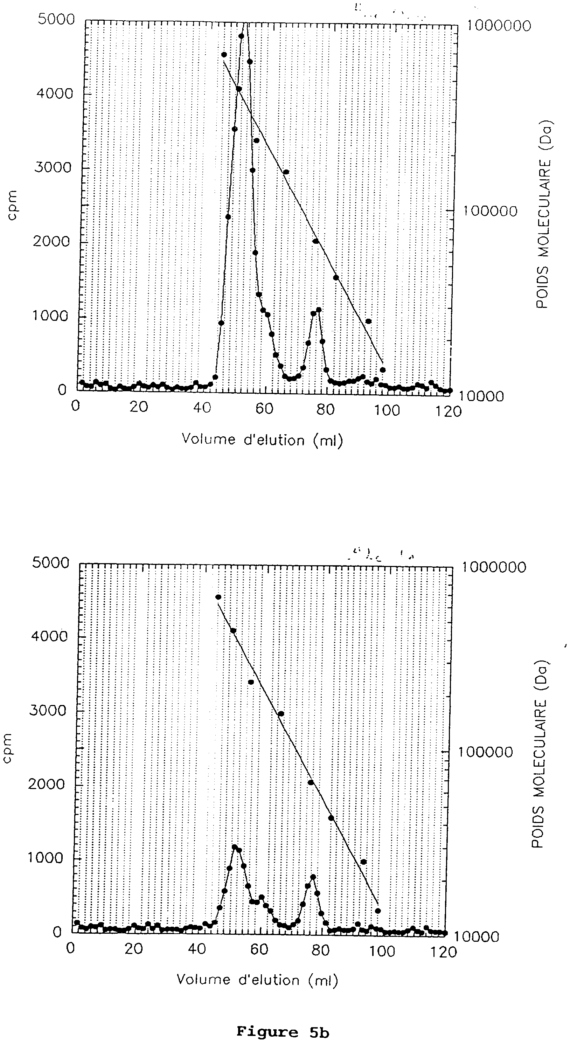

Dans le cas d'un traitement par les quatre anticorps AH65+AH64+BE4+BE8, seuls trois anticorps peuvent se lier simultanément à l'IL-6. L'injection de ce mélange doit entraîner la formation de deux types de complexes trivalents avec l'IL-6, les uns associant les anticorps AH64, AH65 et BE4 , les autres AH65, BE4 et BE8. Le comportement pharmacocinétique observé est très proche de celui décrit précédemment. En particulier, la vitesse initiale d'élimination de l'IL-6 est proche de celle de l'IL-6 libre. On peut détecter 10 minutes après l'injection des complexes trivalents résiduels, qui ont complètement disparu 1 heure après l'injection (figure 5a). Le fractionnement du plasma confirme le phénomène d'élimination préférentielle des complexes trivalents (figure 5b).

**4. Liaison préférentielle des complexes immuns trivalents aux récepteurs pour les immunoglobulines de la lignée monocytaire P388D1.**

La voie d'élimination et par suite la cinétique d'élimination d'un complexe immun in-vivo dépendent de la capacité de ce complexe à être reconnu par les cellules portant des récepteurs pour les immunoglobulines. Les complexes immuns trivalents formés avec l'IL-6 par trois anticorps capables de lier trois épitopes distincts de la molécule cible sont capables de se fixer préférentiellement sur les récepteurs pour les immunoglobulines. Cette propriété distingue les complexes trivalents des complexes monovalents et divalents. Les expériences citées en exemple ci-dessous démontrent la différence de comportement pharmacodynamique que l'on peut attendre d'un mélange constitué d'au moins trois anticorps liant trois épitopes distincts de la molécule cible. En même temps, ces expériences fournissent la base d'un essai in-vitro simple à mettre en oeuvre pour sélectionner une combinaison d'anticorps présentant les propriétés requises pour l'utilisation in-vivo.

Culture de la lignée murine P388 D1 :

La lignée murine d'origine monocytaire P388D1 est disponible auprès de l'ATCC (ATCC N° TIB63). La lignée est maintenue en culture continue dans un incubateur humidifié à 37°C sous 5 % de $CO_2$ (incubateur Narco) en milieu RPMI 1640 supplémenté en acides aminés non essentiels, glutamine 2mM, pyruvate de sodium 1mM, pénicilline 50 UI/ml, streptomycine 50 UI/ml et sérum de veau foetal à 10 % du volume final. Tous ces réactifs ont été fournis par Flow Laboratories, Ecosse. La lignée a été cultivée en flacons de culture de 250 ml (Falcon). La lignée est adhérente. Lorsque les cellules sont à confluence, la culture est traitée par une solution de trypsine-EDTA (Flow) conformément aux protocoles publiés et la suspension cellulaire obtenue est remise en culture après dilution au cinquième. Les expériences de liaison ont été réalisées sur des suspensions cellulaires obtenues à partir de cultures venant d'atteindre la confluence.

## Test de liaison de l'IL-6 radiomarquée sur la lignée P388D1

En premier lieu, une suspension cellulaire de la lignée P388D1 est préparée à partir de cultures à confluence par traitement ménagé à la trypsine. La suspension cellulaire ainsi obtenue est lavée trois fois en tampon phosphaté salin isotonique (PBS), par centrifugation. Les cellules sont reprises en un tampon phosphate salin isotonique pH 7.50 contenant 10 g/l d'albumine bovine (Boehringer) et 5mM d'azothydrure de sodium (Sigma) (désigné comme un tampon PBS/BSA/N3), et la densité cellulaire est finalement ajustée à 1 106 cellules par ml. Pour le test de liaison proprement dit, la suspension cellulaire est distribuée dans une plaque de culture à 96 puits à fond rond (Falcon, Beckton-Dickinson), à raison de 100 $\mu$l/puits, soit $10^5$ cellules par puits. Dans chaque puits, sont ajoutés successivement 150 $\mu$l d'une solution d'IL-6 radiomarquée diluée à 3 $10^6$ cpm/ml en PBS/BSA/N3 puis 100 $\mu$l d'une solution du ou des agents à tester dilué (s) dans le même tampon. Le mélange est ainsi incubé pendant 2 heures à 37°C dans un incubateur (Narco), sous agitation. Puis la radioactivité liée aux cellules est séparée de la radioactivité libre selon un protocole précédemment décrit (Dower S.K. et al, Biochemistry 20:6326, 1981). On prélève dans chaque puits 150 $\mu$l de la suspension cellulaire, qui ont été déposés sur 200 $\mu$l d'un mélange de phtalates (1,2 volume dibutyl-phtalate 90 % pour 1 volume di(2-éthyl-hexyl)phtalate 97 % dans un tube polypropylène de 500 $\mu$l (Eppendorf). Les tubes sont centrifugés à 10000 G pendant 20 secondes dans une centrifugeuse Beckmann de type Microfuge. Après centrifugation, on prélève 50 $\mu$l de la phase aqueuse qui contient la radioactivité libre, puis le tube est découpé aux ciseaux pour obtenir le culot cellulaire qui contient la radioactivité liée. Les échantillons sont alors comptés dans un compteur gamma (Packard), et les résultats sont corrigés des différents facteurs de dilution pour obtenir la fraction d'IL-6 liée dans les différentes conditions testées. Pour chaque condition, l'essai est réalisé en double.

Deux expériences ont été réalisées selon le protocole décrit précédemment (test de liaison à P388D1).

## Expérience N° 8 :

L'IL-6 radiomarquée est incubée avec la suspension cellulaire en présence d'un, deux, trois ou quatre anticorps anti-IL-6. Les anticorps utilisés dans cette expérience sont BE4, BE8, AH64 et AH65. Dans toutes les conditions, la concentration totale d'anticorps est 3,57 $\mu$g/ml (concentration finale dans le puits). Lorsque plusieurs anticorps sont présents, ils sont à même concentration : 1,8 $\mu$g/ml pour chaque anticorps pour deux anticorps présents, 1,2 $\mu$g/ml pour trois anticorps présents, 0.9 $\mu$g/ml pour quatre anticorps. Les résultats de l'expérience sont donnés au tableau C. En l'absence d'anticorps, l'IL-6 radiomarquée ne se lie pas à la lignée P388D1. On constate que la liaison de l'IL-6 radiomarquée est beaucoup plus importante lorsque trois anticorps sont présents. Cet effet n'est observé que pour trois anticorps capables de lier simultanément l'IL-6. Ainsi, lorsque BE8, BE4 et AH64 sont utilisés, le niveau de liaison correspond à celui observé pour une combinaison de deux anticorps, ce qui correspond à la compétition entre BE8 et AH64.

L'expérience réalisée démontre la fixation préférentielle des complexes immuns trivalents sur les récepteurs pour la partie constante des immunoglobulines.

TABLEAU C

| MELANGE D'ANTICORPS | ANTICORPS LIES/LIBRES |
|---|---|
| AH65 | 4.5 |
| AH64 | 5.5 |
| BE8 | 5.49 |
| BE4 | 2.91 |
| AH65/AH64 | 8.28 |
| AH65/BE8 | 9.04 |
| AH65/BE4 | 8.08 |
| AH64/BE8 | 5.32 |
| AH64/BE4 | 9.25 |
| BE8/BE4 | 13.48 |
| AH65/AH64/BE8 | 7.3 |
| AH65/AH64/BE4 | 38.8 |
| BE8/BE4/AH65 | 43.12 |
| BE8/BE4/AH64 | 12.27 |
| BE8/BE4/AH64/AH65 | 28.39 |

**Expérience N° 9 :**

L'IL-6 radiomarquée est incubée avec une combinaison de trois anticorps anti-IL-6 (AH64, AH65 et BE4) à différentes concentrations en anticorps total. A partir d'une solution contenant au total 3.57 µg/ml d'anticorps, soit 1.2 µg/ml pour chaque anti-IL-6, on réalise une gamme de dilution de 2 en 2 jusqu'à 110 ng/ml d'anticorps total. Cette gamme de dilution est testée dans l'essai de liaison sur P388D1. Les résultats sont donnés au tableau D. Sur toute la gamme de concentration testée, la combinaison AH64+AH65+BE4 permet de précipiter efficacement l'IL-6 radiomarquée.

Ce résultat indique que la fixation préférentielle des complexes immuns trivalents peut avoir lieu pour de faibles concentrations en anticorps anti-IL-6.

TABLEAU D

| CONCENTRATION EN ANTICORPS (µg/ml) | LIES/LIBRES AH65/AH64/BE4 | LIES/LIBRES AH65/BE4/BE8 |
|---|---|---|
| 3.57 | 28.36 | 29.91 |
| 1.78 | 43.12 | 46.71 |
| 0.89 | 49.63 | 44.75 |
| 0.44 | 34.59 | 46.94 |
| 0.22 | 37.9 | 42.57 |
| 0.11 | 41.85 | 38.5 |
| 0.00 | 2.15 | 2.13 |

Exemple de préparation pharmaceutique :

On a préparé des ampoules renfermant, l'une :
7 mg d'anticorps lyophilisé AH65
7 mg d'anticorps lyophilisé BE 4
7 mg d'anticorps lyophilisé BE 8
l'autre : eau pour préparation injectable 2 ml.

**CONCLUSIONS** :

1) Il est possible pour une molécule de type cytokine d'identifier trois anticorps monoclonaux capables de lier simultanément cette molécule et de former ainsi des complexes immuns trivalents. Il est possible d'identifier des combinaisons satisfaisant à ce critère et comprenant au moins un anticorps inhibant l'activité biologique de la molécule cible.

2) Lorsqu'un anticorps monoclonal reconnaissant une molécule de type cytokine est injecté à un animal, les complexes immuns anticorps-cytokine qui en résultent sont éliminés de la même manière que l'anticorps. La durée de vie de la cytokine liée à un anticorps étant alors dix fois supérieure à celle de la cytokine libre, l'injection de l'anticorps provoque donc une accumulation de la cytokine sous forme de complexe immun monovalent, ce qui compromet l'efficacité du traitement.

3) L'injection simultanée de trois anticorps anti-cytokine reconnaissant trois épitopes distincts permet la formation de complexes qui sont éliminés rapidement de la circulation. Dans ce cas, il n'y a pas d'accumulation de la cytokine dont on cherche à inhiber l'activité. Ce résultat n'est obtenu spécifiquement que pour des complexes trivalents ; les complexes divalents sont éliminés comme les complexes monovalents. Il est donc avantageux pour inhiber in-vivo l'activité d'une molécule de type cytokine d'utiliser une combinaison de trois anticorps liant cette molécule par trois épitopes distincts.

4) Les complexes immuns trivalents obtenus par réaction avec une molécule de type cytokine de trois anticorps reconnaissant des épitopes distincts de cette molécule sont fixés préférentiellement par les récepteurs pour la partie constante des immunoglobulines (FcR). Le résultat n'est obtenu spécifiquement que pour des complexes trivalents, les complexes divalents ne se fixent pas de façon significative au FcR. Le résultat confirme l'avantage que l'on a à utiliser in-vivo une combinaison de trois anticorps qui peut permettre l'élimination de la molécule cible via le système réticulo-histiocytaire.

5) L'utilisation d'une combinaison de deux anticorps anti-cytokine également inhibiteurs reconnaissant deux épitopes distincts de cette cytokine permet de diminuer la dose totale d'anticorps nécessaire pour bloquer l'activité de la molécule cible. Il est donc avantageux d'utiliser in-vivo une combinaison d'anticorps bloquants, et à fortiori selon les résultats précédemment décrits une combinaison de trois anticorps reconnaissant trois épitopes distincts de la molécule cible et comprenant au moins deux anticorps bloquants.

6) Il est avantageux pour l'efficacité de la préparation que les anticorps utilisés aient une grande affinité pour l'antigène, de sorte que les complexes immuns résultant formés soient très stables et que la forme trivalente soit prépondérante. La cinétique d'association avec un antigène protéique en solution varie peu d'un anticorps à l'autre. Au contraire les complexe immuns formés peuvent avoir une plus ou moins grande stabilité. Il est avantageux que la demi-vie de la liaison antigène-anticorps soit très supérieure à la demi-vie de l'anticorps dans la circulation. Ce critère est satisfait pour des anticorps d'affinité supérieure à $10^9$ et de préférence $10^{10}$ l/M.

**Revendications**

1. Kit thérapeutique anti-médiateur protéique à durée de vie courte et de taille suffisante pour disposer d'au moins trois sites anticorps, caractérisé en ce qu'il renferme au moins trois anticorps indépendants reconnaissant trois épitopes distincts dudit médiateur protéique, au moins un desdits trois anticorps bloquant l'activité biologique dudit médiateur protéique.

2. Kit thérapeutique selon la revendication 1, caractérisé en ce que le médiateur protéique est une cytokine.

3. Kit thérapeutique selon la revendication 1 ou 2, caractérisé en ce que les anticorps sont monoclonaux.

4. Kit thérapeutique selon l'une des revendications 1 à 3, caractérisé en ce que les anticorps sont de type IgG.

5. Kit thérapeutique selon l'une des revendications 1 à 4, caractérisé en ce que deux desdits au moins trois anticorps bloquent l'activité biologique du médiateur protéique.

6. Kit thérapeutique selon l'une des revendications 1 à 5 caractérisé en ce que les anticorps ont une affinité supérieure à $10^9$ litre/mole.

7. Kit thérapeutique selon l'une des revendications 1 à 6, caractérisé en ce que les anticorps sont partiellement humanisés.

8. Kit thérapeutique selon l'une des revendications 1 à 7, caractérisé en ce qu'il comprend les anticorps suivants :
   - AH64, AH65 et BE4 ou
   - AH65, BE4 et BE8 ou
   - AH64, AH65, BE4 et BE8.

9. Procédé de préparation d'un kit tel que défini à la revendication 1, caractérisé en ce que l'on associe au moins trois anticorps reconnaissant trois épitopes distincts dudit médiateur protéique, au moins un desdits au moins trois anticorps bloquant l'activité biologique dudit médiateur protéique.

10. Procédé selon la revendication 9, caractérisé en ce que l'on associe au moins trois anticorps selon les combinaisons suivantes :
    - AH64, AH65 et BE4 ou
    - AH65, BE4 et BE8 ou
    - AH64, AH65, BE4 et BE8 .

11. Compositions pharmaceutiques, caractérisées en ce qu'elles comprennent un kit tel que défini à l'une des revendications 1 à 8.

Figure 1a

Figure 1b

IL6 : △ ; IL6 + AH65: ○ ; IL6 + AH64 : ▽

LOG (POURCENTAGE DE DOSE INJECTE/GR)

PLASMA

FOIE

POUMON

COEUR

REINS

INTESTIN

RATE

TEMPS (HEURES)

Figure 2a

Figure 2b

Figure 3a

**Figure 3b**

Figure 4a

Figure 4b

Figure 5a

**Figure 5b**

EP 0 635 271 A1

**Office européen des brevets**

# RAPPORT DE RECHERCHE EUROPEENNE

Numero de la demande

**EP 94 43 0005**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | WO-A-92 01472 (CELLTECH LIMITED) <br> * revendications * <br> --- | 1-7,9,11 | A61K39/395 |
| D,A | EP-A-0 430 193 (CENTRE RÉGIONAL DE TRANSFUSION SANGUINE & BIOTEST PHARMA GMBH) <br> * le document en entier * <br> --- | 1-11 | |
| A | EP-A-0 410 813 (FUJIREBIO INC. & T.KISHIMOTO) <br> * revendications * <br> --- | 1-11 | |
| A | RESEARCH IN IMMUNOLOGY, <br> vol.143, no.7, 1992, PARIS, FRANCE <br> pages 774 - 776 <br> B. KLEIN ET AL. 'Clinical applications of IL-6 inhibitors.' <br> * le document en entier * <br> ----- | 1-11 | |

|  | DOMAINES TECHNIQUES RECHERCHES (Int.Cl.6) |
|---|---|
|  | A61K |

Le présent rapport a été établi pour toutes les revendications

| Lieu de la recherche | Date d'achèvement de la recherche | Examinateur |
|---|---|---|
| LA HAYE | 20 Octobre 1994 | Nooij, F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

24